(19) Europäisches Patentamt
European Patent Office
Office européen des brevets

(11) Numéro de publication: **0 487 619 B1**

(12) **FASCICULE DE BREVET EUROPEEN**

(45) Date de publication de fascicule du brevet: **12.10.94**

(51) Int. Cl.5: **A61K 37/16**

(21) Numéro de dépôt: **90913211.0**

(22) Date de dépôt: **16.08.90**

(86) Numéro de dépôt internationale :
**PCT/FR90/00613**

(87) Numéro de publication internationale :
**WO 91/02539 (07.03.91 91/06)**

# (54) PROCEDE D'OBTENTION, A PARTIR DE LA CASEINE BETA, DE FRACTIONS ENRICHIES EN PEPTIDES A ACTIVITE BIOLOGIOUE ET LES FRACTIONS PEPTIDIOUES OBTENUES.

(30) Priorité: **16.08.89 FR 8910931**

(43) Date de publication de la demande:
**03.06.92 Bulletin 92/23**

(45) Mention de la délivrance du brevet:
**12.10.94 Bulletin 94/41**

(84) Etats contractants désignés:
**AT BE CH DE DK ES FR GB IT LI LU NL SE**

(56) Documents cités:
**EP-A- 0 033 686**

**Colloque INSERM, vol. 174, (2nd forum on peptides) John Libby Eurotext Ltd 1989 J. Leonil et al**

**Patent Abstracts of Japan, vol. 12, no.159,**

**Patent Abstracts of Japan, vol. 9, no.5**

(73) Titulaire: **INSTITUT NATIONAL DE LA RECHERCHE AGRONOMIOUE (INRA)**
**147, rue de l'Université**
**F-75341 Paris Cédéx 07 (FR)**

(72) Inventeur: **LEONIL, Joelle**
**7, rue des Polieux**
**F-35000 Rennes (FR)**
Inventeur: **NAU, Françoise**
**La Locquenais**
**F-35580 Guichen (FR)**
Inventeur: **MOLLE, Daniel**
**Le Closbourde**
**F-35310 Chavagne (FR)**
Inventeur: **MAUBOIS, Jean, Louis**
**La Barre Guibourg**
**F-35740 Pace (FR)**

(74) Mandataire: **Phélip, Bruno**
**c/o Cabinet Harlé & Phélip**
**21, rue de La Rochefoucauld**
**F-75009 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne un procédé de préparation, à partir de la caséine β, de fractions peptidiques qui renferment des activités physiologiques.

La caséine β représente environ 34% en poids de la caséine entière de lait de vache, soit 8,5 g/l environ, et 60 à 65% de la caséine entière du lait de chèvre. La caséine β du lait de vache est formée d'une chaîne peptidique unique, ne contenant ni cystéine, ni glucides, mais riche en proline (ALAIS, Science du Lait, 1984, 107-199). La caséine β peut être préparée à partir du lait, de caséinate ou de phosphocaséinate natif selon le procédé décrit dans le brevet français FR A 2 592 769 ou par d'autres technologies: chromatographie sur résines échangeuses d'ions (MERCIER et al., Bull. Soc. Chim. Biol. 50, 521-530) ou solubilisation à froid au pH isoélectrique (MANSON et ANNAN, Biochem. Biophys., 1971, 145, 16-26).

Les fractions peptidiques qui sont visées selon l'invention sont:

(A) la fraction I-25,

(B) des fractions précurseurs de la β-casomorphine, laquelle représente la fraction 60-66, ces précurseurs étant des fragments couvrant la zone 33-97 de la molécule de caséine β, ainsi que

(C) la fraction 177-183,

l'apparition de ces fractions étant suivie en chromatographie liquide haute performance en phase inverse (RP-HPLC) sur une colonne C18 en tampon d'élution phosphate de sodium 20 mM et gradient d'acétonitrile de 0 à 60%, avec détection à 214 nm. (LEONIL J. et al., Le Lait, 1988, 68(3), 281-294.

(A) La peptide I-25 contient un enchaînement original en sérines phosphorylées (positions 15, 17, 18 et 19 de la chaîne peptidique) et est qualifié de ce fait de phosphopeptide. Ce phosphopeptide, comme les autres phosphopeptides contenus dans les autres caséines du lait, possède la propriété d'augmenter l'absorption d'oligoéléments, calcium notamment, à travers la paroi intestinale, en les maintenant à l'état soluble au sein de la lumière intestinale. Cet effet des phosphopeptides issus des caséines sur l'absorption du calcium est connu depuis 1950 (MELLANDER, Acta Soc. Med. Upsal, 1950, 55, 247-255).

Le lait et ses dérivés sont en effet considérés comme la source principale d'apport du calcium à l'organisme humain. Ce concept recouvre, en fait, deux notions, celle d'un apport quantitatif (1200 mg de Ca par litre de lait) et celle d'une assimilabilité élevée (85% du calcium contenu dans la poudre de lait absorbé contre 22 à 72% du calcium présent dans les végétaux de la ration - RENNER dans Milk and Dairy Products in Human Nutrition, pages 190-233). L'efficacité de l'absorption intestinale du calcium du lait a été longtemps attribuée au seul lactose selon un mécanisme intervenant au niveau des premiers segments de l'intestin (duodénum et jéjunum): la biotransformation de ce sucre en acide lactique abaisserait la barrière d'énergie des cellules épithéliales et conduirait à une pénétration du calcium sous forme de lactate ou de sels complexes solubles. Les résultats récemment acquis tendent à montrer qu'au contraire, une partie non négligeable de l'absorption intestinale du calcium du lait se ferait selon un autre mécanisme dans lequel interviendraient des segments peptidiques originaux des caséines: les phosphopeptides.

Les caséines $alpha_s$, B et K contiennent, en effet, des enchaînements de sérines phosphorylées qui confèrent à ces protéines un pouvoir chélatant très marqué vis-à-vis des éléments alcalinoterreux ($Ca^{++}$ et $Mg^{++}$) et des oligo-éléments. Cette aptitude est d'autant plus marquée que le taux de phosphorylation est élevé, c'est-à-dire que le pouvoir séquestrant des différentes caséines se situe comme suit: $alpha_{s2}$ > $alpha_{s1}$ > β > K. La répartition des sites phosphoséryle n'est pas uniforme; ainsi, pour les caséines $alpha_s$ et β, la majeure partie de ces sites se situe respectivement entre les résidus 46 à 68 et entre les résidus I à 20. Le pouvoir chélatant élevé de ces phosphopeptides (CPP) fait que la totalité du phosphore et du calcium micellaires leur est associée. Il en résulte que ces enchaînements jouent un rôle essentiel dans la stabilité des micelles de caséine et dans les mécanismes gouvernant la formation du gel lors de la coagulation du lait. Cependant, les caractéristiques physico-chimiques très particulières de ces CPP ont également amené à penser qu'ils intervenaient dans l'absorption intestinale des minéraux et des oligo-éléments.

Ces CPP sont, en effet, retrouvés dans la lumière intestinale chez des rats ayant reçu un régime à base de caséine sous une forme identique à celle obtenue lors de la digestion enzymatique "in vitro" de cette même caséine. A partir d'expérimentations menées sur des boucles duodénales ligaturées, LEE et al. (Agric. Biol. Chem., 1979, 43, 2009-2011) ont, non seulement confirmé que les CPP accroîssaient la solubilité intraluminale du calcium, mais aussi démontré une élévation de l'absorption intestinale de cet élément. Il a par ailleurs été mis en évidence que cette absorption ne nécessitait pas l'intervention de la vitamine D. Bien qu'obtenus chez le poulet, ces résultats précisaient les observations faites auparavant par MELLANDER et OLSON (Bol. Med. Hosp. Infanc. Mex., 1956, 13, 243-246) sur deux groupes d'enfants

rachitiques ou non, recevant des hydrolysats trypsiques de caséine. Plus récemment, cette augmentation de la biodisponibilité du calcium par les CPP a été particulièrement illustrée par GERBER et JOST (Calcif. Tissue Int., 1986, 38, 350-357). La mise en contact de ces segments de caséine avec des extraits embryonnaires d'os: fémur, tibia et métatarse, induisait la minéralisation de ces organes. Cette propriété était perdue si on procédait à une déphosphorylation enzymatique des CPP. En ce qui concerne le mécanisme biochimique dans lequel interviendraient les CPP lors de l'absorption intestinale du calcium, l'hypothèse la plus probable serait, selon SATO et al. (J. Nutr. Sci. Vitaminol., 1986, 32, 67-76), que les CPP inhiberaient la précipitation des sels de calcium au niveau de l'intestin grêle et ainsi favoriseraient une absorption de type passif au niveau de l'iléon.

L'isolement des phosphopeptides peut se faire soit à partir du lait, des caséinates, soit à partir d'une des caséines, le plus souvent la caséine $\beta$. Après hydrolyse trypsique, les séquences phosphopeptidiques sont agrégées par mise en contact avec du calcium et du phosphate minéral. La séparation et la purification de l'agrégat se fait par ultrafiltration sur membrane ou par gel filtration ou encore par chromatographie d'échange d'ions. Les préparations commerciales se caractérisent par une teneur élevée en acide glutamique (28 g p. 100), en sérine (9 g p. 100), en calcium (7g p. 100) et en phosphore (3,6 g p. 100) et une très grande solubilité (200 g/l). Elles peuvent chélater des oligo-éléments en quantité élevée sans que soit altérée leur solubilité.

(B) Les fractions contenant, dans leur séquence, le fragment 60-66 appelé $\beta$-casomorphine peuvent être considérées comme des précurseurs de la $\beta$-casomorphine et être qualifiées de pro-$\beta$-casomorphines. La $\beta$-casomorphine est décrite comme une exorphine, équivalent exogène des endorphines (opioïdes endogènes dont les plus connus sont les Met- et Leu-enképhalines), dont elle aurait toutes les propriétés de régulation du transit des électrolytes (effet antidiarrhée), d'induction du sommeil, de suppression de la douleur ou d'immunomodulation (E. PAROLI, Wld Rev. Nutr. Diet., Vol. 55, pages 58-97. KARGER, BASEL, 1988).

Le relargage de peptides à activité opioïde lors de l'hydrolyse digestive de protéines alimentaires a été mis en évidence lors de recherches portant sur les liens de causalité existant entre la psychose schizophrénique et le régime alimentaire. En effet, nombre d'observations réalisées jusqu'à la fin des années 60 avaient montré l'étroite corrélation chez les personnes prédisposées à la schizophrénie, entre un régime à base de céréales et le syndrome de la maladie coeliaque, une entéropathie affectant les sujets consommant de la gliadine (une des protéines du gluten). La suspension des symptômes liée à l'élimination de la gliadine du régime et au contraire, leur récurrence quand des protéines de gluten, de soja ou de lait étaient administrées aux patients, le déclenchement de modifications du comportement chez des animaux ayant reçu par voie orale ou par injection intracérébrale les protéines du gluten ont constitué un ensemble de faits qui ont amené les chercheurs à supposer un effet psychotoxique direct d'un peptide du gluten.

La similarité des symptômes observés avec ceux reliés à la sécrétion de peptides à activité opioïde par le cerveau et la glande pituitaire (les enképhalines et les endorphines), a conduit à rechercher ces opioïdes dans les protéines alimentaires. Ces exorphines ont été mises en évidence dans les hydrolysats pepsiques de gluten de blé, de caséine $alpha_s$ et de caséine $\beta$. Le fait que la totalité des séquences primaires des caséines ait été élucidée, a permis une avance très rapide des recherches portant sur les activités exorphiques présentes dans les protéines laitières. Ainsi BRANTL et al. ont-ils pu rapidement attribuer à l'enchaînement 60-66 de la caséine $\beta$: Tyr - Pro - Phe - Pro - Gly - Pro - Ile l'activité morphinomimétique détectée dans une caséine peptone commerciale. Cette séquence a été dénommée $\beta$-casomorphine l-7. La nécessaire présence des enchaînement Tyr - X - Phe ou Tyr - $X_1$ - $X_2$ - Phe dans les peptides à activité opioïde d'origine endogène ou exogène a permis de montrer l'existence de nombreuses exorphines dans la séquence des protéines du lait produit par les différentes espèces de mammifères. Les caséines $alpha_s$ et $\beta$ apparaissent être les principales sources de ce type de peptides que ce soit dans le lait maternel ou dans le lait bovin (des séquences proches voire identiques ont également été retrouvées dans les caséines de brebis, de bufflesse et de chamelle), bien qu'ils soient également présents dans la séquence des deux principales protéines du lactosérum: l'alpha-lactalbumine et la $\beta$-lactoblobuline (CHIBA H. et al., 1986, dans "Protein tailoringfor food and medical uses", pages 123-153 (Freeney R.E. and Whitaker J.R. eds). Marcel Dekker, N.Y.").

Le résidu Tyr en position N terminale permettrait l'interaction préférentielle avec les récepteurs $\mu$ du cerveau présents en grande majorité au niveau de l'hypothalamus et du thalamus. Ces récepteurs interviennent lors de l'analgésie supraspinale, du relargage de prolactine, de la régulation de la mobilité intestinale et du taux d'acétylcholine. La présence d'un résidu Arg en position N terminale devant une Tyr favoriserait au contraire les interactions avec les récepteurs delta localisés dans la moelle épinière et dans le système limbique, sièges des sensations émotionnelles et des réflexes de mémorisation acquise

(récompense et punition).

La présence d'exorphines issues de la caséine $alpha_s$ et de $\beta$ casomorphines a été détectée par immunochimie dans le duodénum de miniporcs ayant ingéré de la caséine et dans l'intestin grêle d'hommes adultes ayant consommé du lait. Aucun composé réagissant avec des antiexorphines n'a été trouvé à ce jour dans le plasma sanguin des mammifères adultes. Par contre, un précurseur de la $\beta$-casomorphine 7 a été détecté dans le plasma des veaux nouveau-nés. De même, des réactions positives avec des anti $\beta$-casomorphines 7 et 8 ont été trouvées dans des échantillons de plasma sanguin prélevés chez des femmes enceintes et allaitantes alors que ces réactions étaient négatives avec le plasma prélevé chez les hommes et les femmes non enceintes utilisés comme témoins.

Enfin, un composé proche de la $\beta$-casomorphine été mis en évidence dans le plasma sanguin de plusieurs femmes souffrant de psychose post-partum.

Pour revue, ces différents effets sont inclus dans l'article de E. Paroli, Wld Rev. Nutr. Diet., Vol 55, pages 58-97, Karger, Basel 1998.

A partir de cet ensemble d'observations, TESCHEMACHER (Human Lactation 3. Goldman ed., Plenum Press, New York, 1987, 213-225; et Adv. Biosci, 1987, 65, 41-48) a postulé que les $\beta$-casomorphines participeraient à la régulation endocrine de la grossesse et de l'activité sécrétoire de la glande mammaire en modulant la libération d'octocine et de prolactine. Cette hypothèse suppose évidemment que la caséine $\beta$ produite par les cellules mammaires, ou un fragment de cette molécule contenant la $\beta$-casomorphine, soit transférée dans la circulation sanguine au cours de la lactogénèse. Or, de tels transferts cellules mammaires vers le sang ont déjà été mis en évidence pour l'alpha-lactalbumine et pour la caséine Kappa. L'hypothèse de TESCHEMACHER prend également en compte l'augmentation du taux de prolactine plasmatique constatée chez des rats auxquels avait été injectée par voie systémique de la $\beta$-casomorphine. Il apparaît donc que, lors des modifications physiologiques de l'organisme qui accompagnent la grossesse et, par extension, la reproduction des mammifères, il y ait, en quelque sorte, suppléance ou renforcement des optiates endogènes du cerveau par des composants à activité morphino-mimétiques sécrétés par la glande mammaire.

A partir d'expérimentation menées chez des chiens avec les différentes $\beta$-casomorphines, il a été mis en évidence un mécanisme complexe de régulation de l'appétit et de la prise de nourriture dans lequel intervenaient ces exorphines en opposition avec les endorphines. L'ingestion d'un repas test comportant du saccharose et additionné de $\beta$-casomorphines ou seulement de caséopeptone multipliait en effet le taux d'insuline plasmatique par 6 ou 7 (NIETER et al, Diabetologia, 1981, 21, 309). L'ingestion de repas similaires ou dans lesquels la caséopeptone était remplacée par du lait frais provoquait l'apparition dans le plasma sanguin d'un composant réagissant avec une antistomatostatine. L'accroissement post-prandial d'insuline et de somatostatine était supprimé si de la naloxone, inhibiteur spécifique des opioïdes était ajoutée au repas ou bien si des endorphines telles que la Leu et la Met-enképhaline étaient injectées par voie intraveineuse.

L'activité analgésique des exorphines provenant de la caséine $\beta$ a été très largement étudiée. Sur la base du test "dit" du retrait de la queue de souris après pincement, l'injection intra-cérébroventriculaire des différentes $\beta$-casomorphines a permis de quantifier leur activité anti-douleur non seulement entre elles, mais aussi par rapport à la morphine. Par exemple, la $\beta$-casomorphine l-5 est 10 à 20 fois moins active que la morphine, bien qu'il suffise de 0,06 à 2 $\mu$mole par rat pour obtenir un effet analgésique appréciable; par contre des analogues, tels que la $\beta$-casomorphine l-4 $NH_2$ appelée encore morphiceptine, modifiés par substitution des acides aminés L en position 2 et 4 par des acides aminés D ou par de l'acide pipécolique, seraient 10 fois plus actifs que la morphine. La formation au niveau de l'intestin grêle de la morphiceptine semble hautement probable puisqu'une réaction positive est observée avec un anticorps spécifique et que des enzymes d'amidation existent dans le tractus gastro-intestinal et dans la sécrétion pancréatique. Le transfert à l'homme des observations faites chez le rat est loin d'être évident, mais il est permis de relier l'effet analgésique des exorphines laitières aux observations faites quotidiennement sur le comportement après tétée ou après absorption de biberon des nouveau-nés. Le calme et l'induction du sommeil post-prandiaux peuvent tout à fait résulter de la formation intestinale d'exorphines. Une conclusion semblable est formulée par STURNER et CHANG (Pediatr. Res., 1988, 23, 4) qui ont rapproché le contenu très élevé en $\beta$-casomorphine (9500 nmole/g) et en morphiceptine (l,4 nmole/g) des laits infantiles prédigérés avec la réduction significative des pleurs et l'accroissement du sommeil chez les enfants recevant ces laits.

Par ailleurs, les travaux de HAUTEFEUILLE et al. (Am. J. Physiol.; Gastrointes. Liver Physiol., 250 G 92 - G 97, 1986) et TOME et al. ont mis en évidence que les $\beta$-casomorphine I - 7 et morphiceptine) augmentaient, comme les endorphines, l'absorption des électrolytes sur des tests in vitro réalisés en chambre de Ussing. En conséquence, ils auraient une action anti-diarrhéique potentielle.

(C) Le peptide 177-183 est décrit comme un inhibiteur de l'enzyme de conversion de l'angiotensine I en

angiotensine II (Angiotensin Converting Enzyme ou ACE). Comme tous les inhibiteurs de cette enzyme, ce peptide serait potentiellement un anti-hypertensif puisqu'il permettrait d'abaisser la tension artérielle.

L'examen systématique des séquences des peptides inhibiteurs de l'ACE ayant montré qu'ils avaient tous l'enchaînement Pro-Pro, Ala-Pro ou Ala-Hyp à leur COOH terminal, on a recherché l'existence d'un tel enchaînement dans les peptides issus de l'hydrolyse trypsique de la caséine. A l'aide de deux tests in vitro basés sur l'inactivation de la bradykinine (composé vasodilatateur-hypotenseur provoquant également les contractions de l'utérus de rate et de l'iléon de rat) par l'ACE, on a identifié trois séquences peptidiques inhibitrices de l'ACE dont l'une, qualifiée de CEI $B_7$, a l'enchaînement Ala-Val-Pro-Tyr-Pro-Gln-Arg, et constitue le segment 177-183 de la caséine $\beta$.

Une autre activité potentielle du peptide 177-183 serait, selon MIGLIORE-SAMOUR et JOLLES (Experimenta, 1988, 44, 158-163), une action d'immunomodulation favorisant la production d'anticorps.

Par analogie avec les activités immunomodulantes de peptides produits par les bactéries, PARKER et al. (Eur. J. Biochem., 1984, 145, 677-682) se sont posés la question de l'éventuelle existence dans le premier aliment de l'être humain, à savoir le lait, de fractions d'activité similaire. Leur démarche très finaliste s'appuyait sur le constat de la résistance aux infections bactériennes du nouveau-né nourri au sein, à travers les âges, avant tout développement de la pharmacothérapie. A l'aide de deux tests in vitro: la sécrétion d'anticorps hémolytique contre les hématies de mouton par les cellules de la rate de souris - la phagocytose des héméties de mouton par les macrophages des cellules péritonéales de la souris, ils ont mis en évidence une activité immunostimulante dans l'hydrolysat trypsique de caséine humaine. Le peptide responsable a été purifié et identifié. Il s'agissait de la séquence Val-Glu-Pro-Ile-Pro-Tyr correspondant au segment 54-59 de la caséine $\beta$ humaine. Cette séquence a été synthétisée et administrée parallèlement au peptide naturel par voie intraveineuse à des souris adultes, à des doses de l'ordre de 0,5 mg/kg. Les deux peptides augmentaient significativement la résistance à une infection Klebsiella pneumoniae, mais le peptide naturel était nettement plus efficace. Il est à noter que la séquence 54-59 se retrouve en grande partie dans la séquence à activité exorphique de la caséine $\beta$ humaine ($\beta$-casomorphine I-7). Par ailleurs, la même activité immunostimulante a été mise en évidence pour la séquence 63-68 de la caséine $\beta$ bovine (MIGLIORE-SAMOUR et JOLLES, 1988 selon la publication précitée), à savoir: Pro-Gly-Pro-Ile-Pro-Asn.

Le but visé selon l'invention est atteint par le fait qu'on réalise l'hydrolyse par une enzyme protéolytique (hydrolyse trypsique) de la caséine $\beta$ en milieu salin, cette hydrolyse étant alors une hydrolyse ménagée, qui provoque l'apparition dans le milieu d'un précipité facilement séparé de la phase soluble.

L'intérêt de cette séparation réside dans le fait que la phase soluble ne contient presque que le phosphopeptide (peptide I-25) et les séquences contenant a $\beta$-casomorphine, la fraction enrichie en peptide à activité antihypertensive (fraction 177-183) étant obtenue par une nouvelle hydrolyse du précipité par une enzyme protéolytique (hydrolyse trypsique).

Le procédé de l'invention met en oeuvre les propriétés inattendues et simultanées de libération spécifique du peptide I-25 et d'insolubilisation partielle du "reste" de la molécule de caséine $\beta$, en milieu salin, notamment de force ionique élevée, lors de l'hydrolyse trypsique de la caséine $\beta$.

L'hydrolyse trypsique de la caséine $\beta$ est décrite dans la littérature par les chercheurs ayant déterminé la séquence primaire (enchaînement des acides aminés) de cette protéine (RIBADEAU DUMAS et al., 1970, Eur. J. Biochem., 14, 451-459). La formation des peptides I-25 et 177-183, liée à la spécificité de la trypsine, est indiquée, mais au pH de l'hydrolyse (8,5), il n'y a aucune mention de la formation d'un précipité, et cette hydrolyse n'est pas réalisée en milieu de force ionique élevée. Il en est de même pour les travaux les plus récents sur la caractérisation analytique des peptides formés lors de l'hydrolyse trypsique de la caséine $\beta$: CARLES et al. (J. Dairy Res., 1986, 595-600); LEADBEATER et WARD (J. of Chromatography, 1987, 397, 435-443); PETRILLI et al. (Int. J. Peptide Protein Res., 1987, 29, 504-508). En revanche, ces auteurs décrivent la formation d'un précipité lorsque le pH de l'hydrolysat est abaissé à 4,6. Cette précipitation se faisant après hydrolyse totale de le caséine $\beta$, il est évident que la composition en peptides du précipité et celle du surnageant sont très différentes des produits de la présente invention. L'originalité de la présente invention est le fait que la présence d'un milieu de force ionique élevée permet d'effectuer une protéolyse limitée dans la première phase d'hydrolyse, avec insolubilisation concomitante de certains produits d'hydrolyse.

La formation d'un précipité lors de l'hydrolyse trypsique de la caséine $\beta$ est évoqué par CHRISTEN-SEN (Arch. Biochem. Biophys., 1954, 128-137), mais cette hydrolyse n'est pas réalisée en milieu salin, et l'auteur formule l'hypothèse que le précipité serait un polymère, ce qui conduit l'homme du métier à se désintéresser de ce précipité, dont la composition est inconnue. PETERSON et al. (J. Amer. Chem. Soc., 1958, 80, 95) mentionnent également la formation d'un précipité au cours de l'hydrolyse trypsique de la caséine $\beta$. Cette hydrolyse, contrairement à celle effectuée selon la présente invention, se fait en absence de sel.

LEONIL et al. (Lait, 1988 (68 (3), 281-294 et Colloque INSERM "Second Forum on Peptides" Vol. 174, pp. 65-68, John Libbey Eurotext Ltd, 1988) ont décrit la cinétique d'hydrolyse trypsique de la caséine β et indiquent que la formation du précipité dépend de la concentration initiale en caséine β et du rapport E/S. Ce précipité serait constitué des fragments 29-209, 106-209, 108-209, mais leur étude n'est pas réalisée en milieu salin.

CARLES et al. (FEBS Letters, 1988, 229, N° 2, 265-272) décrivent l'hydrolyse chymotrypsique et thermolysique de la caséine β en présence de sel, notamment KCl. Ils ont mis en évidence le fait que la présence de sel limite l'hydrolyse, mais ils n'ont pas utilisé la trypsine qui a une spécificité d'action différente de celle de la chymotrypsine et de la thermolysine. De plus, ces auteurs ne décrivent pas la formation d'un précipité. L'hydrolyse en milieu salin de la caséine entière par la neutrase, l'alcalase, la papaïne et la chymosine, a également été décrite par SANOGO et al. (Science des Aliments, 1987, 7, 385-398), mais elle ne concerne ni la caséine β, ni la trypsine.

La préparation des peptides selon le procédé de l'invention est extrapolable industriellement. En effet, ce procédé utilise des techniques séparatives à même de traiter des quantités élevées de solution.

On connaît plusieurs méthodes permettant une préparation en laboratoire des peptides biologiques cités. A titre d'illustration pour la préparation de la fraction I-25, on peut citer celle publiée par PETERSON et al. (J. Am. Chem. Soc., 1958, 80, 95-99), où, après une digestion de la caséine β par la trypsine, le précipité formé dans le milieu réactionnel est éliminé par centrifugation. Le surnageant résultant contenant le phosphopeptide I-25 est acidifié à pH = 4,7, débarrasse à nouveau d'un précipité qui se forme à ce pH. Le surnageant riche en phosphopeptide est purifié sous forme de sel de baryum par addition successives de chlorure de baryum et précipitation sous l'action de l'éthanol.

Cette méthode est résumée dans le schéma ci-après:

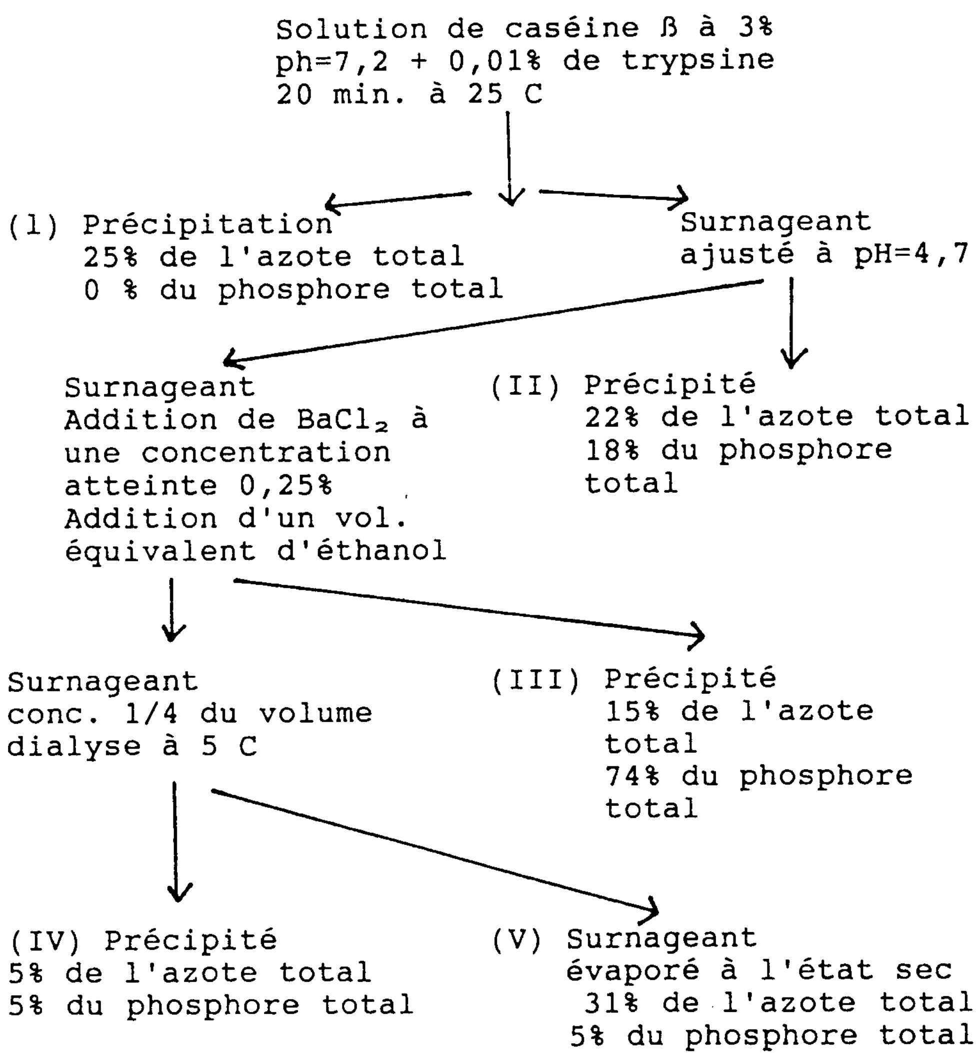

Afin d'extraire le phosphopeptide I-25, ces auteurs passent par l'intermédiaire d'un sel de baryum, forme sous laquelle le phosphopeptide est plus facilement isolé, en raison de son insolubilité dans l'éthanol. La purification ultérieure du phosphopeptide passe par deux étapes chromatographiques, la première visant à éliminer le baryum, la seconde permettant l'obtention du phosphopeptide sous forme pure. Ce procédé, impliquant différentes étapes de précipitation du phosphopeptide sous forme de sel de baryum (méthode délicate où le rendement n'est pas très élevé) et de méthodes chromatographiques, s'avère long et peu rentable. Des méthodes peu différentes de la précédente ont été décrites par MANSON et al. (Arch. Biochem. Biophys., 1971, 145, 16-26), MIKKANEN et al. (J. Nutr. 1980, 110, 2141-2148).

Une modification de cette méthode a été introduite par GERBER et al. (Calcif. Tissu Int., 1986, 38, 350-357), où la précipitation au chlorure de baryum est remplacée par une chromatographie sur échangeur d'ions. La même démarche méthodologique est suivie par SATO et al. (J. Nutri. Sci. Vitaminol., 1986, 32, 67-76); ces auteurs ajoutent une étape chromatographique supplémentaire par perméation de gel. On connaît également le brevet français N° 80-02281, dans lequel est décrite la préparation des phosphopeptides résultant de l'hydrolyse par la pancréatine de la caséine entière.

Dans aucune de ces méthodes, il n'est fait mention de l'utilisation de sel au cours de l'hydrolyse trypsique de la caséine $\beta$, qui est la base de la présente invention. L'effet du sel est d'induire une dégradation très limitée de la molécule de caséine $\beta$ de départ avec libération rapide du phosphopeptide I-25 et ce, avec peu de produits contaminants. La récupération du phosphopeptide I-25 est facilitée par la précipitation qui se produit dans le milieu réactionnel, due à l'insolubilité du ou des restes de la molécule de caséine $\beta$.

Cette dégradation limitée, dont la persistance est fonction de la concentration en sel utilisée, est compatible avec une exploitation technologique. Elle permet d'obtenir d'une manière simple un surnageant dans lequel le phosphopeptide représente environ 20% du matériel peptitique total. Le phosphopeptide peut être ensuite purifié jusqu'à l'homogénéité avec des techniques chromatographiques connues de l'homme du métier. Le procédé selon la présente invention se distingue des autres méthodes connues dans la mesure où est obtenu, grâce à la protéolyse limitée, un enrichissement en phosphopeptide de la fraction soluble ne nécessitant pas l'étape d'extraction au chlorure de baryum.

Simultanément à la formation du phosphopeptide, sont libérés des précurseurs du fragment 60-66 de la caséine $\beta$ appelé $\beta$-casomorphine. Quelques travaux de la littérature mentionnant la formation éventuelle de précurseurs de la $\beta$-casomorphine sans proposer toutefois de méthodes de purification. A titre d'illustrations, on peut citer les études de RIBADEAU DUMAS et al. (Eur. J. Biochem., 1970, 14, 451-459) REIMERDES (J. Dairy Research, 1979, 46, 223-226), LEONIL et al (Le lait, 1988, 68 (3), 281-294), PETRILLI et al. (Int. J. Peptide Protein Res., 1987, 29, 504-508). Dans la présente invention, la plupart des précurseurs de la $\beta$-casomorphine sont relargués dans le milieu lors de la protéolyse trypsique limitée de la caséine $\beta$, en présence de sels.

Le peptide 177-183 est purifié par MARUYAMA et al (Agric. Biol. Chem., 1985, 49 (5), 1405-1409) à partir d'une hydrolyse trypsique de la caséine entière. Trois étapes chromatographiques sont utilisées pour la purification du peptide 177-183 qui sont successivement de la fabrication sur gel, de l'échange d'ions et à nouveau de la filtration sur gel.

Le peptide 177-183, comme décrit dans la présente invention, est obtenu au cours d'une nouvelle hydrolyse trypsine du précipité mentionné précédemment, ce dernier résultant de la protéolyse limitée de la molécule de caséine $\beta$ de départ. Ce précipité a l'avantage d'être une fraction appauvrie en certaines séquences de la molécule de caséine $\beta$ à initiale.

Les préparations de tous les peptides cités dans l'invention peuvent facilement être mises en oeuvre à l'échelle industrielle et toutes les fractions observées peuvent être valorisées. De plus, ledit procédé partant de substance dite naturelle (la caséine du lait de mammifère), met en oeuvre des techniques physiques de séparation après utilisation d'une enzyme physiologique (la trypsine), pour conduire à l'obtention de peptides dits naturels, qualificatif utilisé en opposition aux peptides obtenus par la voie de la synthèse organique ou du génie génétique. Ces deux dernières voies exigent que des opérations de purification très poussées et donc très coûteuses soient menées sur les peptides préparés, en raison des risques de toxicité liés à la présence éventuelle de substances chimiques ou organiques contaminantes. Tel n'est pas le cas des peptides préparés selon l'invention, puisque les substances contaminantes (peptides issus de la molécule de caséine $\beta$) auront au pire un rôle de nutriment. Un autre inconvénient de l'approche par synthèse chimique est le coût de production trop élevé des peptides pour un usage en nutrition.

Il est à noter également que des séquences semblables à celles décrites dans le présent procédé se retrouvent dans la caséine $\beta$ du lait de vache, de chèvre et de brebis. On peut donc envisager d'utiliser ces trois sources de caséine $\beta$ pour la conduite du procédé selon l'invention.

La présente invention a donc d'abord pour objet un procédé d'obtention, à partir de la caséine β, d'un hydrolysat partiel enrichi, d'une part, en phosphopeptide ou fraction peptidique I-25, et d'autre part, en précurseurs de la β-casomorphine 60-66 (ces précurseurs étant des fragments couvrant la zone 33-97 de la molécule de caséine β), par hydrolyse de la caséine β par une enzyme protéolytique, caractérisé par le fait qu'on conduit ladite hydrolyse en milieu salin d'une force ionique équivalente à celle d'une solution de sel monovalent comprise entre 0,5 et 4M (notamment entre I et 2M), provoquant l'apparition dans le milieu d'un précipité que l'on sépare de la phase soluble, laquelle constitue l'hydrolysat partiel recherché.

La concentration en sel du milieu a un rôle déterminant sur la rapidité et la faisabilité du procédé. Comme sel, on peut utiliser un sel monovalent, par exemple, le chlorure de sodium ou de potassium, ou un sel divalent, par exemple, le sulfate de sodium ou le sulfate d'ammonium. Le $Na_2SO_4$ a été testé entre 0 et 0,5M, l'optimum s'étant trouvé être à 0,35 M (En termes de force ionique, il s'avère que la force ionique à 0,35 M en $Na_2SO_4$ est équivalente à celle de la solution de NaCl I M).

La séparation du précipité peut être faite soit par centrifugation (par exemple à 15 000g pendant 20 min), soit par microfiltration tangentielle (par exemple, sur membrane d'oxyde de zirconium sur support de carbone M14, présentant un diamètre de pore de 0,14 à 0,2 micromètre, commercialisée par Tech-Sep). La microfiltration peut être suivie d'une diafiltration afin d'épuiser le rétentat en phosphopeptide I-25 et en précurseurs de la β-casomorphine.

Ce procédé est réalisable pour des concentrations en caséine β allant de 0,5 g/l à 80 g/l, avec une séparation des phases soluble et insoluble d'autant plus efficace et rapide que la concentration en caséine β initiale est élevée.

A titre d'enzyme protéolytique, on utilise notamment la trypsine, et, par exemple, la trypsine commercialisée par NOVO INDUSTRIE sous l'appellation "Crystalline Porcine Trypsin 4500 K" ou "5000 K", ou toute autre préparation de trypsine de pureté équivalente. La plasmine, dont la spécificité est très semblable à celle de la trypsine, pourra également être avantageusement utilisée dans le procédé de l'invention. Le rapport enzyme/substrat (p/p) peut varier dans de larges limites; il peut par exemple être de l'ordre de l/100 à l/10 000. La limite supérieure est liée au coût de l'enzyme et la limite inférieure, à la durée de la réaction.

L'hydrolyse enzymatique peut se dérouler dans une gamme de pH allant de 6 à 9 (de préférence 7,5) et à une température de 25 à 45°C (de préférence 40°C). Le temps d'hydrolyse optimal sera fonction des conditions d'hydrolyse; par exemple, à une concentration en Nacl de 2 M, pH = 8 et 40°C, avec la trypsine SERVA traitée au TPCK - L-Tosyl-L-phénylalaninechlorométhylcétone (inhibiteur de la trypsine) (31 U/mg) au rapport enzyme/substrat de 1/1000, l'optimum sera atteint en 40 minutes; cependant, il est possible de travailler à des rapports enzyme/substrat plus faibles.

En fin d'hydrolyse, l'enzyme est détruite par traitement thermique (85°C pendant 20 minutes), ou récupérée par ultrafiltration de la phase soluble sur membrane organique ou minérale à seuil de coupure de l'ordre de 10 000. Cette phase soluble est un hydrolysat partiel enrichi en phosphopeptide I-25 et en précurseurs de la β-casomorphine.

En partant de la phase soluble, on peut ensuite purifier le phosphopeptide par chromatographie d'échange d'ions, après avoir préalablement déminéralisé cette phase soluble par électrodialyse.

On obtient ainsi une fraction fortement enrichie en précurseurs de la β-casomorphine, et une fraction fortement enrichie en phosphopeptide I-25.

On peut également conduire une précipitation par le calcium sur la phase soluble précitée, en faisant suivre par une centrifugation ou une décantation; on obtient ainsi le phosphopeptide ou fraction peptidique I-25 pratiquement pur.

En partant du précipité, il faut le resolubiliser, amener la solution ainsi obtenue à un pH allant de 6 à 9, et procéder à une deuxième hydrolyse par une enzyme protéolytique (hydrolyse trypsique) à une température de 25 à 45°C. Le temps nécessaire pour atteindre l'hydrolyse totale sera fonction des conditions d'hydrolyse; par exemple, avec la trypsine SERVA traitée au TPCK (31 U/mg) au rapport de l/1000, à pH 8 et 40°C, l'hydrolyse totale sera atteinte en I heure.

On peut ensuite concentrer le peptide à activité anti-hypertensive et immunostimulante (fraction 177-183) à partir de cet hydrolysat de trois façons:

- par ultrafiltration, sur membranes organiques ou minérales à seuil de coupure inférieur à 5000, notamment de l'ordre de 1000 ou 3000, on obtient des perméats contenant de 20 à 40% du peptide recherché selon la nature de la membrane utilisée (les pourcentages étant calculés à partir des aires des pics (équivalent en DO à 214 nm) selon l'expression: pourcentage = (100 x aire du peptide considéré)/aire totale des peptides du chromatogramme, d'où il résulte que l'on peut considérer que les pourcentages équivalent sensiblement à des pourcentages en poids de peptide, sachant qu'une droite d'étalonnage a été établie pour chaque peptide considéré);

- par chromatographie d'échange d'ions, en colonne ou en batch, sur résine échangeuse d'anion (Sp-Sephadex[R] C-25 commercialisée par PHARMACIA-LKB) avec un gradient eau/formiate d'ammonium à pH = 7, on obtient une solution plus ou moins enrichie en la fraction 177-183 selon le produit de départ (hydrolysat complet, perméat 1000 ou perméat 3000); en partant du perméat 1000, la pureté pourra avoisiner les 100%;
- par combinaison successive de ces deux techniques, on peut donc améliorer la pureté de la solution de peptide antihypertensif et immunostimulant obtenue et utiliser au mieux la capacité de la résine.

Les peptides recherchés sont donc obtenus, selon les procédés décrits ci-dessus, sous forme de fractions enrichies dont la composition peptidique globale peut être caractérisée par chromatographie haute performance en phase inverse, sur colonne C18 en tampon d'élution phosphate de sodium 20 mM et gradient d'acétonitrile de 0 à 60%, avec détection à 214 nm.

La présente invention porte également sur une fraction peptidique enrichie en peptides à activité biologique, caractérisée par le fait qu'elle renferme la fraction peptidique l-25 ayant une pureté comprise entre environ 18% et environ 27%, et les précurseurs de la β-casomorphine ayant une pureté comprise entre environ 40% et environ 60%.

L'invention porte également sur une fraction peptidique consistant en précurseurs de la β-casomorphine ayant au moins 92% de pureté.

Les exemples suivants illustrent l'invention et sont donnés en liaison avec les figures du dessin annexé qui représentent les profils HPCL des fractions obtenues sur RP-C18-PEP-RPC (Pharmacia) en gradient linéaire de tampon A et B, à un débit de l ml/min.

Légendes:

Tampon A = phosphate de sodium 20 mM pH = 6,75
Tampon B = phosphate de sodium 29 mM, pH = 7,5, en mélange avec 60% d'acétonitrile.

Le gradient va de 0 à 80% de tampon B en 25 min.

I : peptide l-25
II : précurseurs de la β-casomorphine 60-66
III : peptide 177-183.

EXEMPLE 1

12,7g de caséine β bovine lyophilisée (obtenue selon le procédé décrit dans le brevet français N° 86-00325) sont dissous dans un volume tampon TRIS-HCl, 0,l M, pH = 8, contenant du NaCl 2M, de façon que le poids total final soit de 3022,3g. La solution est portée à 40 °C.

Cette solution est soumise à l'action de la trypsine (Trypsine SERVA traitée au TPCK 31 U/mg, de façon que le rapport E/S soit voisin de l/10000. Ceci revient à ajouter l,2 ml d'une solution de trypsine à l mg/ml. L'évolution de l'hydrolyse est suivie par HPCL sur RP-C18-PEP-RPC (Pharmacia) en gradient tampon phosphate 20 mM, pH = 6,75 acétonitrile (de 0 à 60% en acétonitrile). L'hydrolyse est poursuivie pendant 4 h 45 mn, au cours de laquelle un précipité abondant s'est formé. La réaction enzymatique est arrêtée par traitement thermique dans un échangeur tubulaire en inox plongé dans un bain-marie à 88 °C, la solution est ainsi portée à 85 °C en 4 minutes et est maintenue à cette température pendant 25 minutes. Cette inactivation peut aussi être effectuée en batch.

Le mélange réactionnel est centrifugé à 15000g pendant 20 minutes et à 20 °C. En partant de 2747 g d'hydrolysat, le surnageant récupéré a un poids de 2517,9g dans lequel le phosphopeptide l-25 représente environ 20% du matériel peptidique (voir profil HPLC, Figure l).

Légende de la Figure l:

Surnageant de centrifugation de l'hydrolysat trypsique de caséine β en NaCL 2M, dilué au l/8ème - 200 microlitres injectés.

Le culot résultant de cette centrifugation a le profil peptidique montré en Figure 2.

Légende de la Figure 2:

Culot de centrifugation de l'hydrolysat trypsique de caséine β en NaCl 2M, remis en solution, dilué au l/10ème

200 microlitres injectés.

Le culot remis en solution est soumis à une nouvelle hydrolyse trypsique. A titre d'exemple, 840 ml du culot remis en suspension sont hydrolysés par la trypsine dans un rapport voisin de E/S 1/1000 en poids. Très rapidement, la solution devient limpide et l'évolution de l'hydrolyse est suivie en RP-HPLC sur C18-PEP-RPC (Pharmacia) en gradient tampon phosphate 20 mM, pH = 6,75/acétonitrile (de 0 à 60% en acétonitrile). La réaction est arrêtée lorsque la formation du peptide 177-183 est à son maximum, soit après 3 h 30 mn. Le mélange réactionnel est alors porté à 85 ° C pendant 25 minutes (le protocole d'inactivation de l'enzyme est identique à ce qui a été décrit précédemment).

Le milieu réactionnel limpide obtenu est ensuite ultrafiltré sur un module AMICON$^{R}$CH2A, équipé d'une membrane de fibres creuses HIP3-20 (seuil de coupure 3000). L'ultrafiltration est poursuivie pendant 4 heures avec un débit de perméation moyen de 132 ml/heure, ce qui permet un facteur de concentration de 3. Par analyse en RP-HPLC sur colonne C18-PEP-RPC (Pharmacia), on obtient une séparation des différents peptides du mélange qui permet de définir l'obtention du peptide 177-183 dans les proportions suivantes: le fragment 177-183 représente 3,3% de la molécule de caséine $\beta$ (rapporté au nombre d'acides aminés), 5,75% du matériel peptidique présent dans l'hydrolysat du culot de centrifugation et 20,3% du matériel peptidique présent dans le perméat d'ultrafiltration obtenu tel que décrit ci-dessus (Figure 3).

Légende de la Figure 3:

Perméat sur membrane à seuil de coupure 3000 du culot de centrifugation après hydrolyse trypsique, dilué au l/8ème -

200 microlitres injectés.

Bilan matière de l'Exemple 1

| | % MS | % Cendres | Phosphate ppm | $\frac{g.Nx6,38}{kg}$ | $\frac{n}{P}\frac{(mole)}{(mole)}$ |
|---|---|---|---|---|---|
| Solution de caséine ß initiale | 12,84 | 11,04 | 34,68 | 3,71 | 37 |
| Surnageant de centrifugation | 12,77 | 11,19 | 29,77 | 1,62 | 19 |
| Culot de centrifugation redilué | 2,11 | 0,13 | 5,27 | 5,75 | 378 |

$\frac{N}{P}$ (mole/mole) du phosphopeptide 1-25 = 8,00

EXEMPLE 2

13,2g de caséine $\beta$ bovine lyophilisée, auxquels sont ajoutés 350,6g de NaCl, sont dissous dans de l'eau distillée. Le pH du mélange est amené à 8 au moyen d'une solution de NaOH. Le mélange (poids total

final de 3208,5g) est porté à 40 ° C par thermostatation de la cuve.

Cette solution est soumise à l'action de la trypsine (Trypsine TPCK SERVA 31 U/mg) dans un rapport E/S voisin de 1/10 000. La variation du pH du milieu réactionnel est contrôlée et régulée à l'aide d'un pHstat par addition de NaOH 0,5 M. La réaction est poursuivie pendant 5 heures au cours desquelles un précipité abondant s'est formé. La trypsine est inactivée par traitement thermique dans un échangeur tubulaire selon le protocole décrit dans l'Exemple I.

Le mélange réactionnel est centrifugé à 7000 g pendant I heure à 20 ° C (partant de 2785,3g de mélange réactionnel, on récupère 2764,3g de surnageant); le surnageant, analysé par HPLC RP-C18-PEP-RPC, Figure 4, contient principalement les fragments I-25 (20,25% du matériel peptidique présent) et les précurseurs de la $\beta$-casomorphine et le culot obtenu a le même profil analytique que celui montré en Figure 2.

Légende de la Figure 4:

Surnageant de centrifugation de l'hydrolysat trypsique de caséine $\beta$ en NaCl 2M, dilué au I/10ème - 200 microlitres injectés.

Le culot est soumis à l'hydrolyse trypsique après avoir été remis en suspension dans de l'eau distillée ajustée à pH 8 et à 40 ° C. A titre d'indication, 14,54g de culot (poids humide) sont dissous dans un volume d'eau distillée final (1970g) dont le pH est ajusté à 8 au moyen d'un pHstat et la température portée à 40 ° C. La solution est hydrolysée par 8 mg de trypsine (rapport E/S voisin de I/1000 en poids sec). Au cours de l'hydrolyse qui est poursuivie jusqu'à ce que le peptide 177-183 atteigne son maximum de libération, le milieu réactionnel devient limpide. L'évolution de l'hydrolyse est suivie en RP-HPLC (C18-PEP-RPC). Après environ 4 heures, la trypsine est inactivée par traitement thermique selon le protocole décrit dans l'exemple 1.

Le mélange résultant peut être:

1) soit ultrafiltré sur un module plan FILTRON à flux tangentiel (Pharmacia-LKB) équipé de membrane polysulfone (700 $cm^2$) à seuil de coupure 1000. A titre d'indication, en partant de 484 ml (483,8g) d'un hydrolysat tel que décrit précédemment, l'ultrafiltration a un débit entre 180 et 240 ml/heure et est poursuivie jusqu'à un facteur de concentration égal à 2,26. Dans les 234 ml de perméat obtenu, le fragment 177-183 de la caséine $\beta$ représente 39,5% du matériel peptidique (Figure 5), mais seulement 30% du peptide 177-183 initialement mis en oeuvre; le rendement peut atteindre 100%, par diafiltration.

Légende de la Figure 5:

Perméat sur membrane à seuil de coupure 1000 du culot de centrifugation après hydrolyse trypsique, dilué au I/5ème - 200 microlitres injectés.

2) soit ultrafiltré sur le même module plan FILTRON équipé d'une membrane à seuil de coupure 3000. L'ultrafiltration d'un hydrolysat de 500 ml (505,25g) est poursuivie jusqu'à un facteur de concentration égal à 2,63, avec un débit de 600 ml/h. Dans ces conditions, 300 ml (302,7g) de perméat sont obtenus dans lesquels le fragment 177-183 représente 32,3% du matériel peptidique (Figure 6), mais seulement 45,5% du fragment 177-183 initialement mis en oeuvre; le rendement peut atteindre 100% par diafiltration.

Légende de la Figure 6:

Perméat sur membrane à seuil de coupure 3000 du culot de centrifugation après hydrolyse trypsique, dilué au I/5ème - 200 microlitres injectés.

EXEMPLE 3

50,7g de caséine $\beta$ bovine lyophilisée sont dilués dans 5 l de tampon phosphate 0,1 M contenant 2M de NaCl, à pH = 7,5. Cette solution est amenée à 40 ° C et soumise à l'hydrolyse par 5,5 mg de trypsine (TPCK SERVA), dans un rapport E/S voison de 1/10 000.

L'hydrolyse est arrêtée après 5 h 00, pendant lesquelles s'est développée un précipité, par traitement thermique: la solution est plongée dans un bain-marie bouillant dans lequel elle est maintenue 20 minutes à 85 ° C.

L'hydrolysat est soumis à une microfiltration sur un module SFEC équipé d'une membrane tubulaire CARBOSEP M14, de surface 0,l $m^2$, et dont le débit sur eau avant utilisation était équivalent à 618 l/h.$m^2$,

pour une pression d'entrée de 4 bars et une température de 25 ° C.

Une microfiltration est effectuée pendant 25 minutes. Cette microfiltration est suivie d'une diafiltration afin d'épuiser le rétentat. Les performances réalisées au cours de ces deux opérations sont résumées dans le Tableau I. A titre d'exemple, en partant de 5 litres d'hydrolysat, 2,5 litres de microfiltrat contenant 46% du phosphopeptide I-25 initialement présent sont obtenus à un taux de pureté de 18,4% (Figure 7). La diafiltration subséquente permet d'épuiser totalement le rétentat en phosphopeptide I-25.

Légende de la Figure 7:

Microfiltrat sur membrane M14 de l'hydrolysat trypsique de caséine β en NaCl 2M, dilué au l/20ème - 100 microlitres injectés

TABLEAU 1

| Temps après le début de la microfiltration (minutes) | P entrée (bars) | P sortie (bars) | t° (°C) | débit (l/h/m²) |
|---|---|---|---|---|
| 5 | 1,7 | 0,2 | 50 | 76 |
| 10 | 1,4 | 0,6 | " | 76 |
| 20 | 1,3 | 0,5 | " | 65 |
| 25 | 1,5 | 0,5 | " | 78 |
| 30 | 1,5 | 0,5 | " | 88 |
| 38 | 1,6 | 0,5 | " | 91 |
| 45 | 1,6 | 0,5 | " | 92 |
| 55 | 1,6 | 0,5 | " | 92 |

Le schéma d'ensemble de la Figure 8 (sur deux planches du dessin annexé) résume des voies possibles pour l'obtention de fractions enrichies en peptides à activité biologique selon l'invention; sur ce schéma, sont présentés les profils chromatographiques correspondant aux différentes étapes du procédé.

. : phosphopeptide $\beta$-CN (f I-25)

.. : précurseurs de la $\beta$-casomorphine

... : peptide $\beta$-CN (f 177-183).

Les analyses ont été réalisées sur colonne PEP RPC-C18 (Pharmacia) en tampon d'élution phosphate-acétonitrile:

Tampon A = phosphate de sodium 20 mM pH = 6,75

Tampon B = phosphate de sodium 20 mM pH = 7,5, en mélange avec 60% d'acétonitrile.

Le gradient va de 0 à 80% de tampon B en 25 minutes.

La détection est faite à 214 nm, ce qui signifie que l'on suit essentiellement les liaisons peptidiques; on peut donc admettre que le rapport (aire de la protéine ou du peptide recherché x 100/aire totale du chromatogramme) constitue une approche du pourcentage de pureté de la protéine ou du peptide considéré.

Sur la base de cette formule, on peut annoncer, pour les chromatogrammes présentés dans le schéma ci-joint, les taux de pureté suivants:

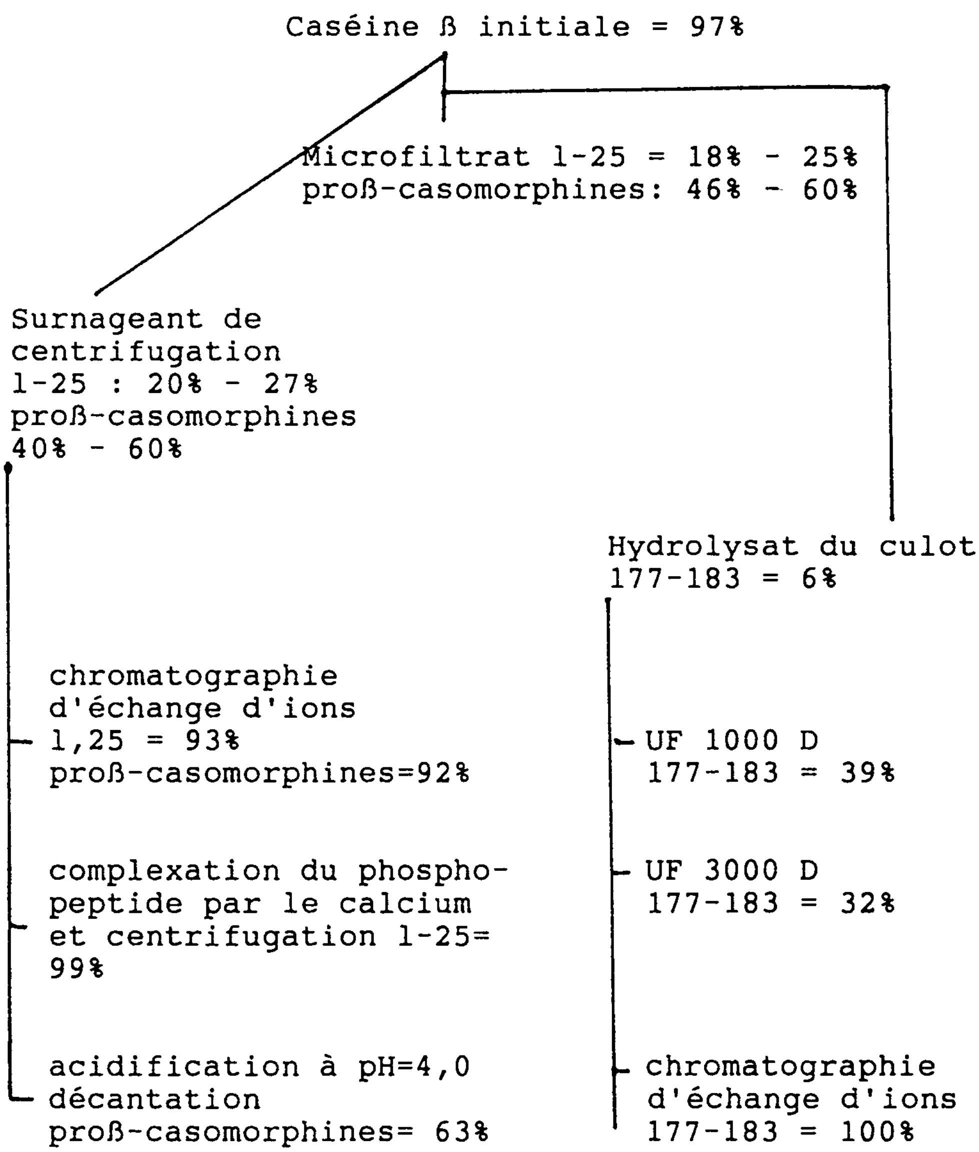


## Revendications

1. Procédé d'obtention , à partir de la caséine $\beta$, d'un hydrolysat partiel enrichi, d'une part , en phosphopeptide ou fraction peptidique 1-25, et d'autre part , en précurseurs de la $\beta$-casomorphine 6O-66 ( ces précurseurs étant des fragments couvrant la zone 33-97 de la molécule de caséine $\beta$) , par hydrolyse de la caséine $\beta$ par une enzyme protéolytique , caractérisé par le fait qu'on conduit ladite

hydrolyse dans un milieu salin concentré de force ionique équivalente à celle d'une solution de sel monovalent comprise entre O,5 et 4 M, provoquant l'apparition dans le milieu d'un précipité que l'on sépare de la phase soluble , laquelle constitue l'hydrolysat partiel recherché .

**2.** Procédé selon la revendication 1, caractérisé par le fait qu'on conduit l'hydrolyse dans un milieu d'une force ionique équivalente à celle d'une solution de sel monovalent entre 1 et 2 M.

**3.** Procédé selon l'une des revendications 1 et 2 , caractérisé par le fait qu'on utilise, comme sel, un sel monovalent , par exemple, le chlorure de sodium ou de potassium, ou un sel divalent , par exemple , le sulfate de sodium, ou le sulfate d'ammonium.

**4.** Procédé selon l'une des revendications 1 à 3, caractérisé par le fait qu'on utilise, comme substance de départ, un produit à base de caséine, ayant une pureté en caséine $\beta$ comprise entre 85 et 9O%, issu notamment du lait de vache , de chèvre ou de brebis .

**5.** Procédé selon l'une des revendications 1 à 4,caractérisé par le fait qu'on conduit l'hydrolyse avec une concentration de départ en caséine $\beta$ comprise entre O,5 g/l et 8O g/l.

**6.** Procédé selon l'une des revendications 1 à 5,caractérisé par le fait qu'on utilise, comme enzyme protéolytique, la trypsine ou la plasmine, et notamment , la trypsine .

**7.** Procédé selon l'une des revendications 1 à 6, caractérisé par le fait qu'on utilise un rapport enzyme/substrat (p/p) de l'ordre de 1/100 à 1/10000.

**8.** Procédé selon l'une des revendications 1 à 7,caractérisé par le fait qu'on conduit l'hydrolyse à un pH allant de 6 à 9 , et notamment à pH 7,5 , et à une température de 25 à 45 ° C, notamment à 4O ° C.

**9.** Procédé selon l'une des revendications 1 à 8,caractérisé par le fait qu'en fin d'hydrolyse, on détruit l'enzyme par traitement thermique ou bien on la récupère par ultrafiltration de la phase soluble sur membrane organique ou minérale à seuil de coupure de 10000.

**10.** Procédé selon l'une des revendications 1 à 9 ,caractérisé par le fait qu'on sépare le précipité obtenu par centrifugation ou par microfiltration tangentielle, ladite microfiltration pouvant être suivie d'une diafiltration afin d'épuiser le rétentat en phosphopeptide 1-25 et en précurseurs de la $\beta$-casomorphine .

**11.** Procédé d'obtention, d'une part , du phosphopeptide ou fraction peptidique 1-25, et , d'autre part , des précurseurs de la $\beta$-casomorphine 6O-66, caractérisé par le fait qu'on conduit une chromatographie d'échange d'ions sur la phase soluble obtenue par le procédé tel que défini à l'une des revendications 1 à 10 , après avoir préalablement déminéralisé cette phase soluble .

**12.** Procédé d'obtention du phosphopeptide ou fraction peptidique 1-25, caractérisé par le fait qu'on conduit une précipitation par le calcium sur la phase soluble obtenue par le procédé tel que défini à l'une des revendications 1 à 10, et l'on fait suivre par une centrifugation ou une décantation .

**13.** Procédé pour l'obtention de la fraction peptidique 177-183 à activité antihypertensive et immunostimulante , caractérisé par le fait qu'on part du précipité tel qu'il est obtenu par le procédé conforme à l'une des revendications 1 à 10 , qu'on le resolubilise, qu'on amène la solution ainsi obtenue à un pH allant de 6 à 9 , et qu'on conduit une hydrolyse par une enzyme protéolytique.

**14.** Procédé selon la revendication 13 , caractérisé par le fait qu'on conduit l'hydrolyse à une température comprise entre 25 et 45 ° C.

**15.** Procédé selon l'une des revendications 13 et 14 , caractérisé par le fait qu'on utilise la trypsine comme enzyme protéolytique.

**16.** Procédé selon l'une des revendications 13 à 15 , caractérisé par le fait qu'on conduit une purification de l'hydrolysat par ultrafiltration sur membranes organiques ou minérales à seuil de coupure inférieur à 5OOO, le perméat étant enrichi en peptide recherché .

**17.** Procédé selon l'une des revendications 13 à 15 , caractérisé par le fait qu'on conduit une purification de l'hydrolysat par chromatographie d'échange d'ions.

**18.** Procédé selon la revendication 16 , caractérisé par le fait qu'on conduit une purification du perméat par chromatographie d'échange d'ions .

**19.** Fraction peptidique enrichie en peptides à activité biologique , caractérisée par le fait qu'elle renferme la fraction peptidique 1-25 ayant une pureté comprise entre environ 18 % et environ 27 % , et les précurseurs de la $\beta$-casomorphine ayant une pureté comprise entre environ 4O% et environ 6O %.

**20.** Fraction peptidique consistant en précurseurs de la $\beta$-casomorphine ayant au moins 92 % de pureté .

**21.** Fraction constituée par le perméat d'ultrafiltration obtenu par le procédé tel que défini à la revendication 16 , sur membrane d'ultrafiltration d'environ 1000 D.

**22.** Fraction peptidique constituée par le perméat d'ultrafiltration obtenu par le procédé tel que défini à la revendication 16 , sur membrane d'ultrafiltration d'environ 3000 D.

**Claims**

**1.** Making method from $\beta$ casein of a partial enriched hydrolysis product, from one hand, in phosphopeptide or 1-25 peptidic fragment and, in the other hand, in 60-66 $\beta$ casomorphine precursors (said precursors being fragments covering the 33-97 area of the $\beta$ casein molecule) by $\beta$ casein hydrolysis with a proteolytic enzyme, characterized in that said hydrolysis is carried out in a concentrated saline medium having an ionic strength corresponding to those of 0.5 to 4 M monovalent salt solution involving in the medium the occurrence of a precipitate which is separated from the soluble phase which is the partial searched hydrolysis product.

**2.** Method according to claim 1, characterized in that the hydrolysis is carried out in a medium having an ionic strength equivalent to those of 1 to 2 M monovalent salt solution.

**3.** Method according to one of claims 1 and 2, characterized in that a monovalent salt is used as salt, for example, sodium chloride or potassium chloride, or a divalent salt is used, for example, sodium sulphate or ammonium sulphate.

**4.** Method according to any one of claims 1 to 3, characterized in that as starting material is used a casein basis product having a $\beta$ casein purity between 85 and 90 % originating particularly from cow milk, goat milk and ewe milk.

**5.** Method according to any one of claims 1 to 4, characterized in that hydrolysis is carried out with a starting $\beta$ casein concentration between 0.5 g/l to 80 g/l.

**6.** Method according to any one of claims 1 to 5, characterized in that as proteolytic enzyme is used trypsin or plasmine and particularly trypsin.

**7.** Method according to any one of claims 1 to 6, characterized in that an enzyme substrate ratio (w / w) from 1:100 to 1:10 000 is used.

**8.** Method according to any one of claims 1 to 7, characterized in that hydrolysis is carried out at a pH between 6 and 9 and particularly at a pH of 7.5 and at a temperature from 25 to 45 °C particularly at 40 °C.

**9.** Method according to any one of claims 1 to 8, characterized in that at the end of the hydrolysis the enzyme is destroyed by thermal treatment or recuperated by ultra-filtration of the soluble phase on a organic or inorganic membrane having a separation threshold of 10 000.

**10.** Method according to any one of claims 1 to 9, characterized in that the precipitate obtained by tangential micro-filtration or centrifugation is separated, said micro-filtration being followed, if desired,

by diafiltration in order to exhaust the retentate in 1-25 phosphopeptide and in β casomorphine precursors.

**11.** Making method from one hand of a phosphopeptide or 1-25 peptidic fragment and on the other hand of 60-66 β casomorphine precursors, characterized in that an ion exchange chromatography is carried out on the soluble phase obtained by the method as defined in any one of claims 1 to 10 after previously made a demineralisation of said soluble phase.

**12.** Making method of phosphopeptide or 1-25 peptidic fragment, characterized in that a precipitation with calcium is carried out on the soluble phase obtained by the method as claimed in any one of claims 1 to 10 which is followed by a centrifugation or decantation step.

**13.** Making method of 177-183 peptidic fragment with anti-hypertension and immunostimulating activity, characterized in that the method begins with the precipitate as obtained with the method according to any one of claims 1 to 10, said precipitate is redissolved and the so obtained solution comprises a pH between 6 and 9 and an hydrolysis with a proteolytic enzyme is carried out.

**14.** Making method according to claim 13, characterized in that the hydrolysis is carried out at a temperature between 25 and 45 °C.

**15.** Method according to one of claims 13 and 14, characterized in that trypsin is used as proteolytic enzyme.

**16.** Method according to one of claims 13 to 15, characterized in that purification of the hydrolysis product is carried out by ultra filtration on an organic or an inorganic membrane with a separation threshold less than 5 000, the permeate being enriched with the searched peptide.

**17.** Method according to one of claims 13 to 15, characterized in that the hydrolysis product purification is carried out by ion exchange chromatography.

**18.** Method according to claim 16, characterized in that a permeate purification is carried out by ion exchange chromatography.

**19.** Peptidic fraction enriched with peptides having a biological activity, characterized in that it comprises the 1-25 peptidic fraction having a purity between about 18% to about 27% and β-casomorphine precursors having a purity between about 40% to about 60%.

**20.** Peptidic fraction consisting in β-casomorphine precursors having at least a purity of 92%.

**21.** Peptidic fraction consisting in the ultra-filtration permeate obtained by the method as claimed in claim 16 on substantially a 1 000 D ultra-filtration membrane.

**22.** Peptidic fraction consisting in the ultra-filtration permeate obtained by the method as claimed in claim 16 on substantially a 3 000 D ultra filtration membrane.

**Patentansprüche**

**1.** Verfahren, ausgehend von Casein β, zur Gewinnung eines Hydrolysats, das partiell angereichert ist an einerseits Phosphopeptid oder der Peptidfraktion 1-25 und andererseits an Vorstufen von β-Casomorphin 60-66 (wobei diese Vorstufen Fragmente sind, die dem Bereich 33-97 des Casein-β-Moleküls entsprechen), durch Hydrolyse von Casein β mittels eines proteolytischen Enzyms, dadurch gekennzeichnet, daß man die Hydrolyse in einer konzentrierten Salzlösung als Medium durchführt, dessen Ionenstärke zu derjenigen der Lösung eines einwertigen Salzes mit einer Konzentration zwischen 0.5 und 4 M äquivalent ist, was in dem Milieu die Erscheinung eines Niederschlags hervorruft, den man von der löslichen Phase abtrennt, welche das gesuchte partielle Hydrolysat bildet.

**2.** Verfahren gemäß Anspruch 1, dadurch gekennzeichnet, daß man die Hydrolyse in einem Medium durchführt, dessen Ionenstärke zu derjenigen der Lösung eines einwertigen Salzes mit einer Konzentration zwischen 1 und 2 M äquivalent ist.

**3.** Verfahren gemäß einem der Ansprüche 1 oder 2, dadurch gekennzeichnet, daß man als Salz ein einwertiges Salz, zum Beispiel Natrium- oder Kaliumchlorid, oder ein zweiwertiges Salz, zum Beispiel Natriumsulfat oder Ammoniumsulfat, verwendet.

**4.** Verfahren gemäß einem der Ansprüche 1 bis 3, dadurch gekennzeichnet, daß man als Ausgangsstoff ein Produkt auf Caseinbasis verwendet, dessen Reinheit an Casein $\beta$ zwischen 85 und 90% liegt, insbesondere aus Kuh-, Ziegen- oder Schafsmilch.

**5.** Verfahren gemäß einem der Ansprüche 1 bis 4, dadurch gekennzeichnet, daß man die Hydrolyse mit einer Ausgangskonzentration an Casein $\beta$ zwischen 0.5 g/l und 80 g/l durchführt.

**6.** Verfahren gemäß einem der Ansprüche 1 bis 5, dadurch gekennzeichnet, daß man als proteolytisches Enzym Trypsin oder Plasmin, und insbesondere Trypsin, verwendet.

**7.** Verfahren gemäß einem der Ansprüche 1 bis 6, dadurch gekennzeichnet, daß man ein gewichtsbezogenes Enzym/Substrat-Verhältnis im Bereich von 1/100 bis 1/10000 verwendet.

**8.** Verfahren gemäß einem der Ansprüche 1 bis 7, dadurch gekennzeichnet, daß man die Hydrolyse bei einem pH-Wert zwischen 6 und 9, und insbesondere bei pH 7.5, und bei einer Temperatur von 25 bis 45 °C, insbesondere bei 40 °C durchführt.

**9.** Verfahren gemäß einem der Ansprüche 1 bis 8, dadurch gekennzeichnet, daß man das Enzym am Ende der Hydrolyse durch thermische Behandlung zerstört oder es durch Ultrafiltration der löslichen Phase auf organischer oder mineralischer Membran mit einer Trennschwelle von 10000 zurückgewinnt.

**10.** Verfahren gemäß einem der Ansprüche 1 bis 9, dadurch gekennzeichnet, daß man den erhaltenen Niederschlag durch Zentrifugieren oder durch tangentielle Mikrofiltration abtrennt, wobei die Mikrofiltration von einer Diafiltration gefolgt sein kann, um zurückgehaltenes Phosphopeptid 1-25 und Vorstufen von $\beta$-Casomorphin auszuwaschen.

**11.** Verfahren zur Gewinnung von einerseits Phosphopeptid oder Peptidfraktion 1-25 und andererseits von Vorstufen von $\beta$-Casomorphin 60-66, dadurch gekennzeichnet, daß man an der durch das Verfahren, wie es in einem der Ansprüche 1 bis 10 definiert ist, erhaltenen löslichen Phase eine Ionenaustauschchromatographie durchführt, nachdem man diese lösliche Phase zuvor entmineralisiert hat.

**12.** Verfahren zur Gewinnung von Phosphopeptid oder Peptidfraktion 1-25, dadurch gekennzeichnet, daß man an der durch das Verfahren, wie es in einem der Ansprüche 1 bis 10 definiert ist, erhaltenen löslichen Phase eine Fällung durch Calcium und anschließend eine Zentrifugation oder Dekantierung durchführt.

**13.** Verfahren zur Gewinnung der Peptidfraktion 177-183 mit antihypertensiver und immunostimulanter Wirkung, dadurch gekennzeichnet, daß man von dem Niederschlag ausgeht, wie er nach dem Verfahren gemäß einem der Ansprüche 1 bis 10 erhalten wurde, ihn wieder in Lösung bringt, die so erhaltene Lösung auf einen pH-Wert zwischen 6 und 9 bringt und eine Hydrolyse durch ein proteolytisches Enzym durchführt.

**14.** Verfahren gemäß Anspruch 13, dadurch gekennzeichnet, daß man die Hydrolyse bei einer Temperatur zwischen 25 und 45 °C durchführt.

**15.** Verfahren gemäß einem der Ansprüche 13 oder 14, dadurch gekennzeichnet, daß man als proteolytisches Enzym Trypsin verwendet.

**16.** Verfahren gemäß einem der Ansprüche 13 bis 15, dadurch gekennzeichnet, daß man eine Reinigung des Hydrolysats durch Ultrafiltration auf organischen oder mineralischen Membranen mit einer Trennschwelle von unter 5000 durchführt, wobei das Permeat mit dem gesuchten Peptid angereichert wird.

**17.** Verfahren gemäß einem der Ansprüche 13 bis 15, dadurch gekennzeichnet, daß man eine Reinigung des Hydrolysats durch Ionenaustauschchromatographie durchführt.

**18.** Verfahren gemäß Anspruch 16, dadurch gekennzeichnet, daß man eine Reinigung des Permeats durch Ionenaustauschchromatographie durchführt.

**19.** Mit biologisch aktiven Peptiden angereicherte Peptidfraktion, dadurch gekennzeichnet, daß sie die Peptidfraktion 1-25 mit einer Reinheit zwischen etwa 18% und etwa 27% sowie die Vorstufen von $\beta$-Casomorphin mit einer Reinheit zwischen etwa 40% und etwa 60% enthält.

**20.** Peptidfraktion, die aus Vorstufen von $\beta$-Casomorphin mit einer Reinheit von wenigstens 92% besteht.

**21.** Fraktion, die aus dem Ultrafiltrationspermeat besteht, wie man es nach dem in Anspruch 16 definierten Verfahren auf einer Ultrafiltrationsmembran mit ungefähr 1000 D erhalten hat.

**22.** Peptidfraktion, die aus dem Ultrafiltrationspermeat besteht, wie man es nach dem in Anspruch 16 definierten Verfahren auf einer Ultrafiltrationsmembran mit ungefähr 3000 D erhalten hat.

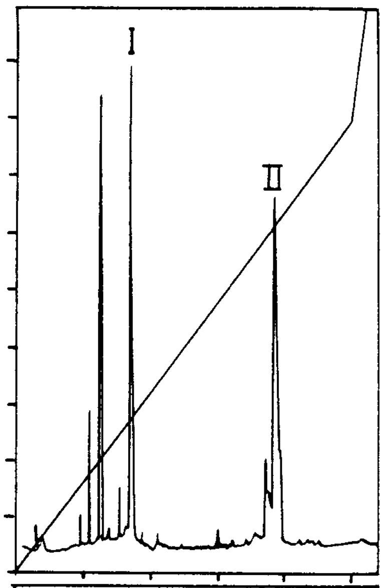


FIG.1

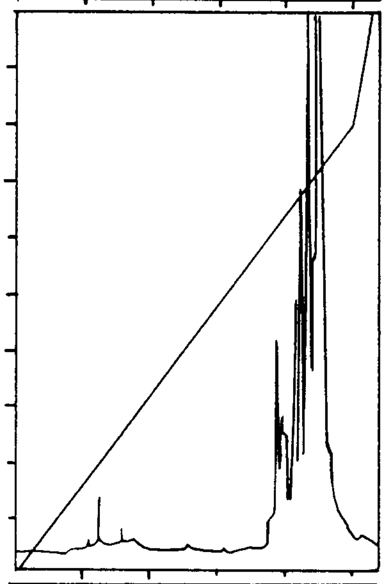

FIG.2

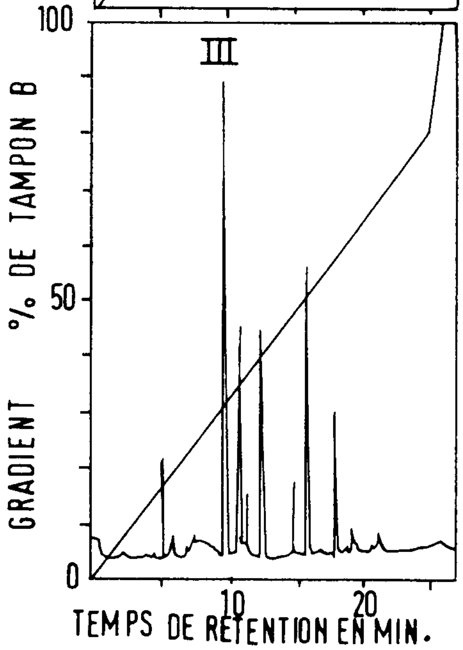


FIG 3

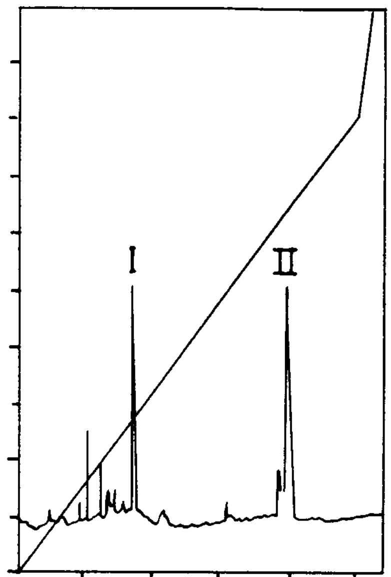


FIG.4

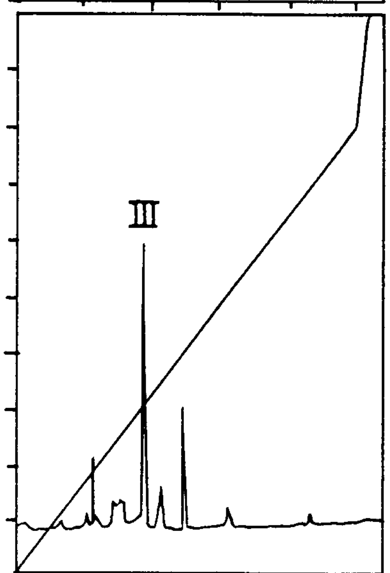


FIG.5

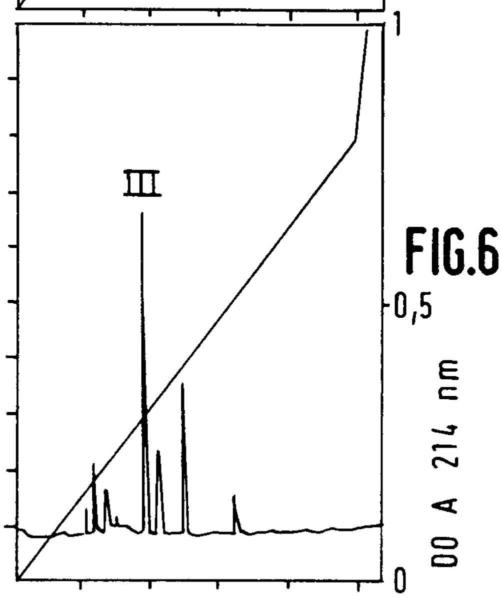


FIG.6

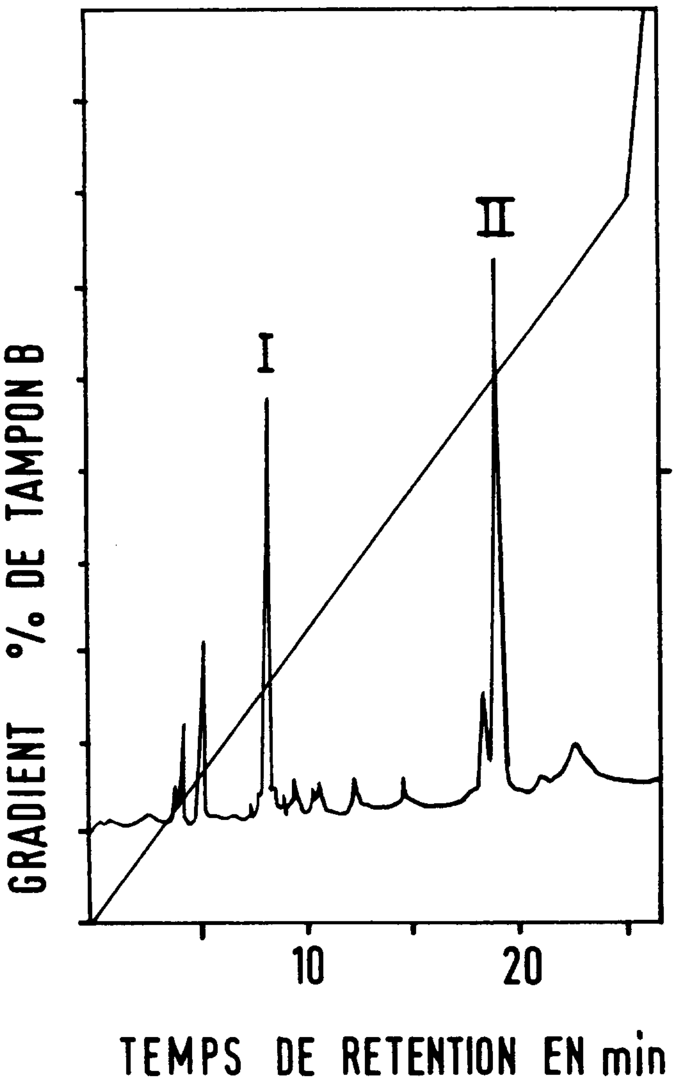
GRADIENT % DE TAMPON B
DO A 214 nm
I
II
10
20
TEMPS DE RETENTION EN min

FIG.7

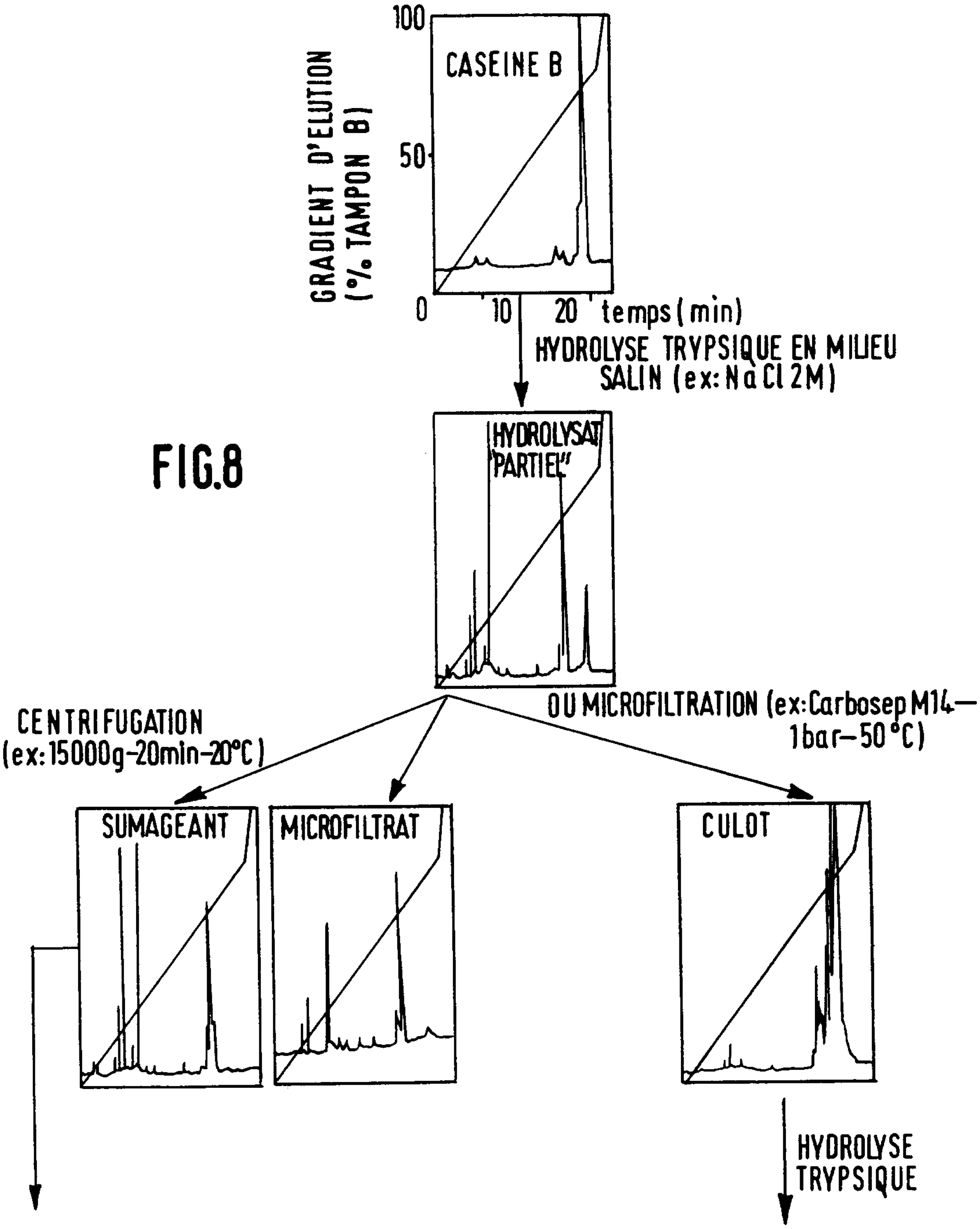
100
50
0
GRADIENT D'ELUTION
(% TAMPON B)
CASEINE B
10
20
temps (min)
HYDROLYSE TRYPSIQUE EN MILIEU
SALIN (ex: NaCl 2M)
HYDROLYSAT
"PARTIEL"
CENTRIFUGATION
(ex: 15000g-20min-20°C)
OU MICROFILTRATION (ex: Carbosep M14-
1bar-50°C)
SUMAGEANT
MICROFILTRAT
CULOT
HYDROLYSE
TRYPSIQUE

FIG.8

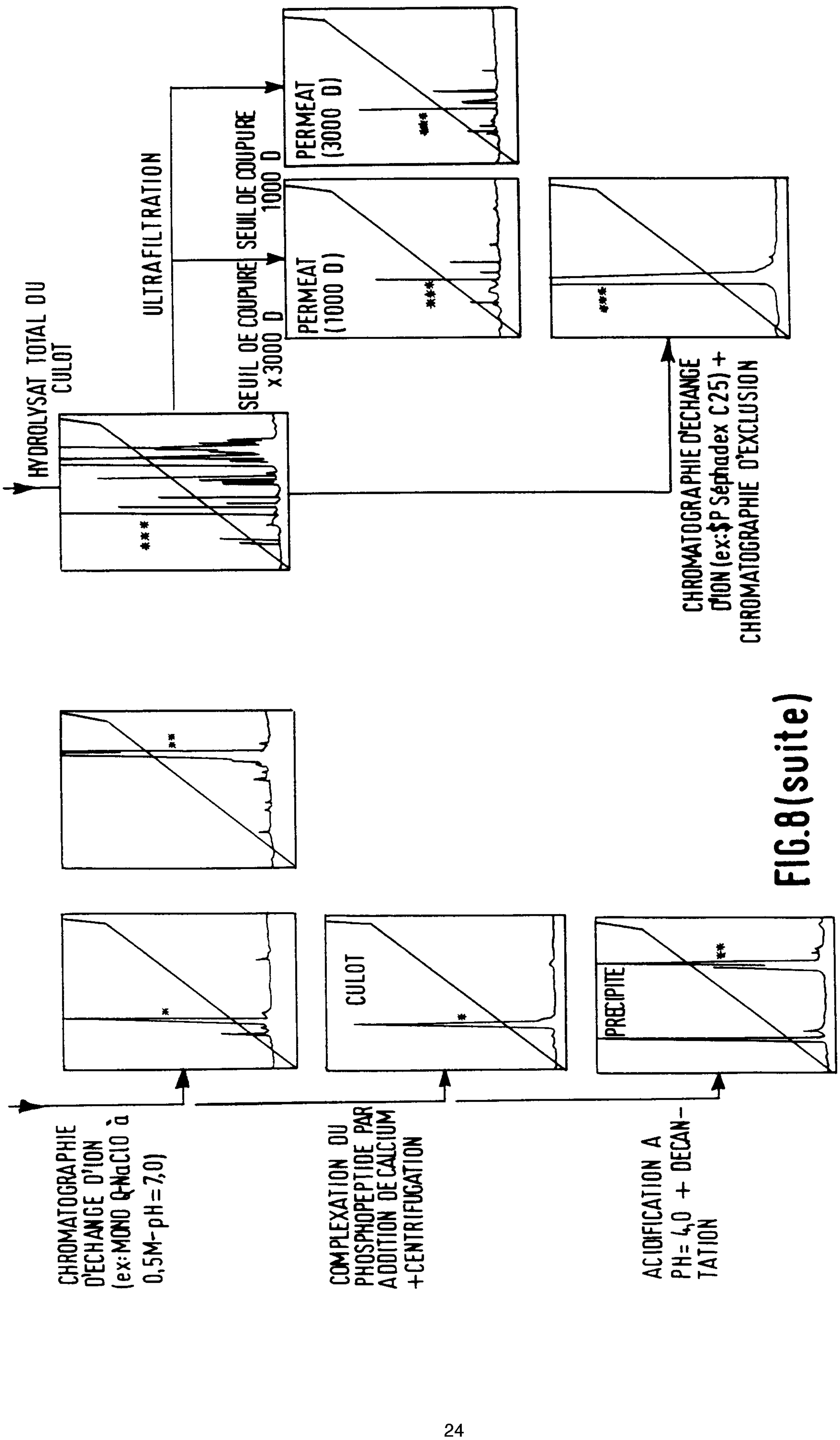
HYDROLYSAT TOTAL DU CULOT
ULTRAFILTRATION
SEUIL DE COUPURE x3000 D
SEUIL DE COUPURE 1000 D
PERMEAT (1000 D)
PERMEAT (3000 D)
CHROMATOGRAPHIE D'ECHANGE D'ION (ex:$P Séphadex C25) + CHROMATOGRAPHIE D'EXCLUSION
CHROMATOGRAPHIE D'ECHANGE D'ION (ex: MONO Q-NaClO à 0,5M-pH=7,0)
COMPLEXATION DU PHOSPHOPEPTIDE PAR ADDITION DE CALCIUM +CENTRIFUGATION
ACIDIFICATION A PH= 4,0 + DECANTATION
CULOT
PRECIPITE

FIG.8(suite)